# EUROPEAN PATENT SPECIFICATION

(11) **EP 2 371 305 B1**
(45) Date of publication and mention of the grant of the patent: **01.02.2012**
(21) Application number: 10158572.7
(22) Date of filing: 31.03.2010
(51) Int. Cl.: A61B 17/16

(54) **Connection between CFK shaft and metal part by wrapping**
Verbindung zwischen einem CFK-Schaft und einem Metallteil durch Umwickeln
Connexion entre une tige CFK et une pièce métallique par emballage

(43) Date of publication of application: 05.10.2011
(73) Proprietor: Stryker Trauma GmbH, 24232 Schönkirchen/Kiel (DE)
(72) Inventor: Giersch, Helge, 24118, Kiel (DE); Stoltenberg, Ingo, 24253, Probsteierhagen (DE)
(74) Representative: Schmitz, Alexander

(56) References cited:
- WO-A1-2009/015672
- US-A- 4 706 659
- US-B1- 7 131 982

## Description

### FIELD OF THE INVENTION

The present invention relates to a reaming device, and in particular to a reaming device providing a reliable connection between a shaft and an interface element.

### BACKGROUND OF THE INVENTION

Intramedullary nailing is the method of choice for the fixation of fractures in long bones, in particular in long extremities. To have a full access to the intramedullary channel, a shaft of a reamer has to be flexible enough in a bending direction to bypass soft tissue and bone curvature, and also has to be rigid enough to convey torsion to the reamer head. Prior art reaming devices have a shaft design consisting of a helix in which residues can be trapped during the reaming procedure, so that the cleaning of the reaming device in hospitals prior to the next usage is complicated, in particular for a sterilisation process. The adequate cleaning of the instrument in hospitals demands a high effort and takes a lot of time. Further, some hospitals are not prepared to clean such critical devices because of the high, effort involved.

In some prior art reaming devices, a helix shaft is replaced by a shaft made of so called nitinol, which is a material having a high degree of elasticity (super elasticity) to provide enough flexibility. Nitinol is an akronym for NIckel TItanium Naval Ordnance Laboratory. Nitinol is the inter-metallic phase NiTi having a regular cubic crystal structure being different of the structure of titanium or nickel. Nitinol comprises about 55% nickel and about 45% titanium. Owing to the fact that the nitinol shaft is made of a single tube, the cleaning effort in the hospital is less exhausting. However, recent investigations have shown that the nitinol material has a catastrophic failure mode. In particular, some reports have pointed out that some breakages in multiple fragments of the nitinol shaft occurred during the reaming process during the intervention process in hospitals. Further, the nitinol material is a very expensive material.

From US 2007/0015107, a root canal instrument having an abrasive coating and method for the production thereof is known, wherein the described root canal instrument has a core of a flexible elastic material having a shape memory, wherein the core furthermore has a coating with abrasive particles, wherein the core is made from a nickel-titanium alloy or from a plastic material, e.g. carbon fibre reinforced plastic material.

CH 668690 describes a probe electrode cable for medical purposes, e.g. electro cardiogram test, using carbon fibre impregnated plastic insulating coating as a cover with a lead coupled to the test equipment.

WO 2009/05672 describes a reaming device with a rod and an interface element connected with a carbon fibre wrapping.

### SUMMARY OF THE INVENTION

It may be seen as an object of the present invention to provide a more reliable reaming device.

The object of the present invention is solved by the subject-matter of the independent claims. Further embodiments are incorporated in the dependent claims.

According to an exemplary embodiment of the invention, a reaming device comprises a shaft with a mounting portion, the mounting portion having an end face and an outer surface; an interface element for mechanical coupling of an external device, the interface element comprises a mounting portion, the mounting portion having an end face and an outer surface; a carbon fiber wrapping; wherein the end face of the shaft mounting portion and the end face of the interface element mounting portion are facing towards each other; wherein the carbon fiber wrapping extends over both, the outer surface of the shaft mounting portion and the outer surface of the interface element mounting portion.

Thus, a shaft of a reamer and an interface element may be reliably coupled and mounted by a carbon fiber wrapping, so that the connection between a shaft and an interface element can be manufactured without building up high internal material tensions, which may lead to an unpredictable material stress. Further, by providing a carbon fiber wrapping over both, the outer surface of the shaft mounting portion and the outer surface of the interface element mounting portion, the total diameter of the reaming device may be kept low, so that the reaming device provides a possibility for narrow spaces.

According to an exemplary embodiment of the invention, the shaft is made of a carbon fiber reinforced material.

Thus, breakages can be avoided, as a carbon fiber reinforced material does not break into multiple fragments like a nitinol shaft. Further by using the same or similar material for the shaft and the wrapping, a reliable connection between the shaft and the wrapping may be established owing to the material compatibility.

According to an exemplary embodiment of the invention, the end face of the shaft mounting portion and the end face of the interface element mounting portion abut to each other.

Thus, the transit portion between the shaft and the interface element can be kept short, so that the shaft device can be designed as a very compact device, in particular close to the interface element.

According to an exemplary embodiment of the invention, the shaft mounting portion and the interface element mounting portion have corresponding outer diameters.

Thus, the carbon fiber wrapping may be wrapped around the outer surface of the shaft mounting portion and the outer surface of the interface element mounting portion without a step, so that sharp chamfers or grooves may be avoided, which chamfers or grooves may lead to a weakened connection owing to the general mechanical weakness of a chamfer.

According to an exemplary embodiment of the invention, the carbon fiber wrapping comprises a mold cover.

Thus, the entire strength of the carbon fiber wrapping can be enforced. Further, the mold cover may seal the carbon fiber wrapping so as to avoid rough and porous surfaces generally leading to problems during the surgical intervention.

According to an exemplary embodiment of the invention, the mold cover is a thermosetting mold cover.

Thus, the connection between the shaft and the interface element by a carbon fiber wrapping can be enforced by the thermosetting process, generally allowing a reliable connection between a shaft and an interface element.

According to an exemplary embodiment of the invention, the interface element comprises a coupling portion for a reamer head as an external device.

Thus, a reamer head or a bore or drill head can be mounted to the interface element.

According to an exemplary embodiment of the invention, the coupling portion comprises a dovetail.

Thus, a reliable connection between a reamer head and the interface element may be established. In particular when providing a dovetail transverse to the longitudinal extension of the interface element, pulling forces as well as pushing forces can be transmitted to or from the reamer head to the reaming device and vice versa. In particular, when providing the reamer head and the interface element with a through bore in a longitudinal direction, a securing or guiding wire can be inserted in order to avoid a lateral movement of the reamer head with respect to the interface element, while maintaining an easy assembling or disassembling when removing the guide wire.

According to an exemplary embodiment of the invention, an outer surface of a coupling portion and the outer surface of the interface element mounting portion are coaxial, wherein the outer surface of the interface element mounting portion steps back over the outer surface of the coupling portion.

Thus, the carbon fiber wrapping can be placed in the stepped back outer surface of the interface element so as to not extend over the outer surface of the coupling portion. This allows for a smooth transition between the coupling portion and the wrapping.

According to an exemplary embodiment of the invention, the carbon fiber wrapping has an outer diameter corresponding to an outer diameter of the coupling portion.

Thus, in particular when providing a step back geometry at the interface element, a smooth total surface can be provided starting from the outer diameter of the coupling portion via the outer diameter of the carbon fiber wrapping, and for example a smooth transition to the shaft. Thus, a smooth surface over the entire interface element and the transition portion to the shaft can be provided without any steps or recesses.

According to an exemplary embodiment of the invention, the outer surface of the interface element mounting portion comprises a recess structure.

Such a recess structure provides a reliable mechanical coupling of the carbon fiber wrapping onto the interface element mounting portion, in particular, when using an impregnated carbon fiber wrapping. Thus, the force transition between the shaft and the interface element is not only based on a surface connection between the carbon fiber wrapping and the interface element, but also by a mechanical recess structure.

According to an exemplary embodiment of the invention, the recess structure comprises a circumferential groove.

Thus, in particular axial forces can be transferred from the shaft to the interface element and vice versa, and a loosing of the interface element with respect to the shaft can be avoided, even if the surface connection between the wrapping and the interface element fails.

According to an exemplary embodiment of the invention, the recess structure comprises a knurl surface.

The knurl surface provides a reliable mechanical coupling between the carbon fiber wrapping and the interface element, and at the same time increases the frictional forces between the shaft and interface element.

According to an exemplary embodiment of the invention, the interface element is made of a titanium alloy.

Thus, in particular a lightweight and medical compatible material can be provided for the interface element.

According to an exemplary embodiment of the invention, the shaft and the interface element each have an elongated through bore, wherein both through bores aligning to each other.

Thus, it is possible to insert a guide wire or securing wire into the aligning through bores. The guide wire may assist for a reliable targeting when reaming, wherein a securing wire may be used to secure a reaming head onto the coupling portion of the interface element.

It should be noted that the above features may also be combined. The combination of the above features may also lead to synergetic effects, even if not explicitly described in detail.

These and other aspects of the present invention will become apparent from and elucidated with reference to the embodiments described hereinafter.

### BRIEF DESCRIPTION OF THE DRAWINGS

Fig. 1 illustrates a shaft and an interface element as separate elements.
Fig. 2 illustrates a cross-sectional view of a shaft and an interface element being mounted together with a carbon fiber wrapping.
Fig. 3 illustrates an end portion of a reaming device with a shaft and an interface element as well as a carbon fiber wrapping.

### DETAILED DESCRIPTION OF EXEMPLARY EMBODIMENTS

Fig: 1 illustrates a shaft 10 having an outer surface 13 and an end face 12. The end portion of the shaft 10 is considered as a shaft mounting portion 11. This shaft mounting portion 11 serves for receiving a carbon fiber wrapping. The end face 12 is substantially perpendicular to the longitudinal direction of the shaft 10, however, it should be understood that the end face 12 may also be inclined or may have a surface structure like for example a waved or a toothed comb structure. The shaft may be made of a carbon fiber material which is capable of carrying torque forces, and at the same time has a certain flexibility, which is required for surgical interventions.

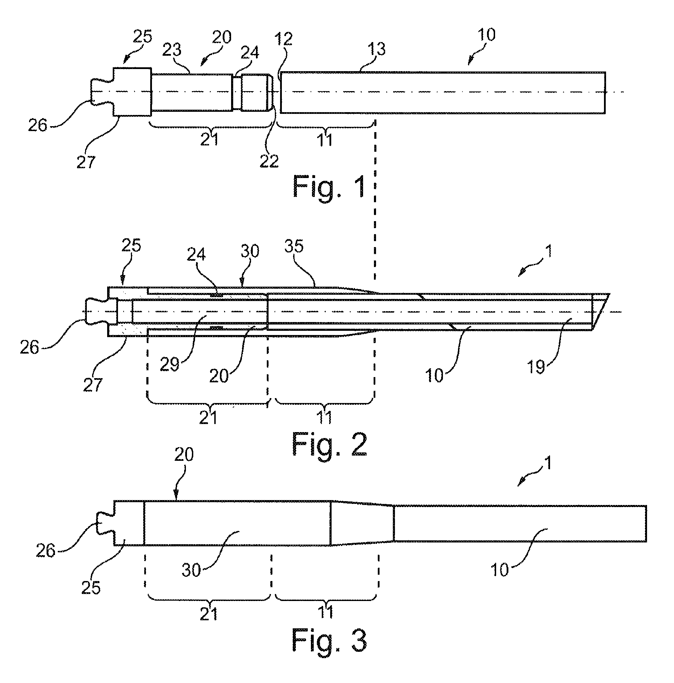

The interface element 20 is also provided with an outer surface 23 and an end face 22. The end face 22 may be substantially perpendicular to the longitudinal extension of the interface element. However, also the end face 22 may comprise a structure, for example corresponding to that of the end face 12 of the shaft in order to have kinds of interfering or mashing end faces 12 and 22. Also the interface element 20 comprises an interface element mounting portion 21 for receiving a carbon fiber wrapping. The interface element 20 further comprises a coupling portion 25, which coupling portion 25 has an outer surface 27. An end portion of the coupling portion 25 may be provided with a coupling element 26, for example a dovetail. However, it should be noted that also any other coupling element 26 may be used. The interface element mounting portion 21 may comprise a surface structure 24, which may be for example a circumferentially extending groove and/or for example a knurl surface (not shown) for a reliable coupling of the carbon fiber wrapping, as will be described with respect to Fig. 2 in further detail.

Fig. 2 illustrates a cross-sectional view of an end portion of a reaming device, wherein the end faces 12 of the shaft 10 and 22 of the interface element 20 abut to each other. The carbon fiber wrapping 30 is wrapped around the outer surfaces 11, 21 of the shaft 10 and the interface element 20, respectively. Thus, a reliable connection between the shaft 10 and the interface element 20 can be established. The combination of a carbon fiber shaft 10 and for example a metal, in particular titanium alloy material for the interface element provide for a reliable combination of a flexible shaft and a form-stable and insensitive interface element for coupling a reamer head (not shown). The surface structure 24 of the mounting portion 21 of the interface element 20 provides for a reliable connection between the carbon fiber wrapping 30 and the surface of the interface element 20. In one case, the shaft 10 is made of a carbon fiber material, the carbon fiber wrapping 30 and the carbon fiber material of the shaft 10 are compatible with each other, so that a reliable connection can be established, even if no surface structure is provided on the mounting portion 11 of the shaft 10. Nevertheless, also the mounting portion 11 of the shaft may be provided for with a surface structure.

As can be seen from Fig. 2, the carbon fiber wrapping 30 has an external dimension/diameter, so that the outer surface 11 of the coupling portion 25 smoothly aligns with the outer surface 35 of the carbon fiber wrapping 30. Thus, a smooth transition between the coupling portion 25 and the carbon fiber wrapping 30 can be established. The carbon fiber wrapping 30 can smoothly transit towards the shaft 10 so as to provide a smooth transition between the carbon fiber wrapping 30 and the surface of the shaft 10.

Both, the shaft 10 and the interface element 20 may be provided with a through bore 19, and 29, respectively. The aligning through bores 19 and 29 provide a possibility to put through a guide wire for a reliable targeting during intervention. Further, a securing wire can be put through the aligning through bores 19 and 29 as well a through bore of a reaming head (not shown) so as to avoid a lateral movement and losing of the reaming head being coupled by for example a dovetail.

Fig. 3 illustrates an outer view of the end portion of the reaming device 1, where it can be seen that the shaft 10, the interface element 20 together with its coupling portion 25, and the carbon fiber wrapping 30 together provide for a smooth surface without any steps or step transitions, so that a smooth total surface can be provided starting from the outer surface of a coupling portion via the outer surface of the carbon fiber wrapping towards an outer surface of the shaft 10.

It should be noted that the term "comprising" does not exclude other elements and that the term "a" or "an" does not exclude a plurality. Also elements described in association with the different embodiments may be combined.

It should be noted that the reference signs in the claims shall not be construed as limiting the scope of the claims.

## Claims

1. Reaming device comprising
a shaft (10) with a mounting portion (11), the mounting portion having an end face (12) and an outer surface (13);
an interface element (20) for mechanical coupling of an external device, the interface element comprises a mounting portion (21), the mounting portion having an end face (22) and an outer surface (23);
a carbon fiber wrapping (30);
wherein the end face (12) of the shaft mounting portion and the end face (22) of the interface element mounting portion are facing towards each other; **characterized in that**
the carbon fiber wrapping extends over both the outer surface (13) of the shaft mounting portion and the outer surface (23) of the interface element mounting portion.

2. Reaming device according to claim 1, wherein the shaft (10) is made of a carbon fiber reinforced material.

3. Reaming device according to any of claims 1 and 2, wherein the end face (12) of the shaft mounting portion and the end face (22) of the interface element mounting portion abut to each other.

4. Reaming device according to any of claims 1 to 3, wherein the shaft mounting portion (11) and the interface element mounting portion (21) have corresponding outer diameters.

5. Reaming device according to any of claims 1 to 4, wherein the carbon fiber wrapping (30) comprises a mold cover (35).

6. Reaming device according to claim 5, wherein the mold cover (35) is a thermosetting mold cover.

7. Reaming device according to any of claims 1 to 6, wherein the interface element (20) comprises a coupling portion (25) for a reamer head as an external device.

8. Reaming device according to claim 7, wherein the coupling portion (25) comprises a dove tail (26).

9. Reaming device according to any of claims 7 and 8, wherein an outer surface (27) of the coupling portion and the outer surface (23) of the interface element mounting portion are coaxial, wherein the outer surface (23) of the interface element mounting portion steps back over the outer surface (27) of the coupling portion.

10. Reaming device according to any of claims 7 to 9, wherein the carbon fiber wrapping (30) has an outer diameter corresponding to an outer diameter of the coupling portion (25).

11. Reaming device according to any of claims 1 to 10, wherein the outer surface (23) of the interface element mounting portion comprises a recess structure (24).

12. Reaming device according to claim 11, wherein the recess structure (24) comprises a circumferential groove.

13. Reaming device according to any of claims 11 and 12, wherein the recess structure (24) comprises a knurl surface.

14. Reaming device according to any of claims 1 to 13, wherein the interface element (20) is made of a titanium alloy.

15. Reaming device according to any of claims 1 to 14, wherein both, the shaft (10) and the interface element (20) each have an elongated through bore (19, 29), the both through bores aligning to each other.

## Patentansprüche

1. Aufreibvorrichtung, umfassend
eine Welle (10) mit einem Anbringungsabschnitt (11), wobei der Anbringungsabschnitt eine Endfläche (12) und eine Außenfläche (13) aufweist;
ein Schnittstellenelement (20) zum mechanischen Ankuppeln einer externen Vorrichtung, wobei das Schnittstellenelement einen Anbringungsabschnitt (21) aufweist, wobei der Anbringungsabschnitt eine Endfläche (22) und eine Außenfläche (23) aufweist;
eine Kohlefaserumwicklung (30);
wobei die Endfläche (12) des Wellenanbringungsabschnitts und die Endfläche (22) des Schnittstellenelementanbringungsabschnitts zueinander hin gekehrt sind, **dadurch gekennzeichnet, dass**
die Kohlenstofffaserumwicklung sowohl über die Außenfläche (13) des Wellenanbringungsabschnitts als auch die Außenfläche (23) des Schnittstellenelementanbringungsabschnitts verläuft.

2. Aufreibvorrichtung nach Anspruch 1, wobei die Welle (10) aus einem kohlefaserverstärkten Material hergestellt ist.

3. Aufreibvorrichtung nach einem der Ansprüche 1 oder 2, wobei die Endfläche (12) des Wellenanbringungsabschnitts und die Endfläche (22) des Schnittstellenelementanbringungsabschnitts aneinander anstoßen.

4. Aufreibvorrichtung nach einem der Ansprüche 1 bis 3, wobei der Wellenanbringungsabschnitt (11) und der Schnittstellenelementanbringungsabschnitt (21) entsprechende Außendurchmesser aufweisen.

5. Aufreibvorrichtung nach einem der Ansprüche 1 bis 4, wobei die Kohlefaserumwicklung (30) eine Gussabdeckung (35) umfasst.

6. Aufreibvorrichtung nach Anspruch 5, wobei die Gussabdeckung (35) eine thermohärtende Gussabdeckung ist.

7. Aufreibvorrichtung nach einem der Ansprüche 1 bis 6, wobei das Schnittstellenelement (20) einen Kupplungsabschnitt (25) für einen Reibahlenkopf als externe Vorrichtung umfasst.

8. Aufreibvorrichtung nach Anspruch 7, wobei der Kupplungsabschnitt (25) einen Schwalbenschwanz (26) umfasst.

9. Aufreibvorrichtung nach einem der Ansprüche 7 oder 8, wobei eine Außenfläche (27) des Kupplungsabschnitts und die Außenfläche (23) des Schnittstellenelementanbringungsabschnitts koaxial sind, wobei die Außenfläche (23) des Schnittstellenelementanbringungsabschnitts über der Außenfläche (27) des Anbringungsabschnitts zurückgestuft ist.

10. Aufreibvorrichtung nach einem der Ansprüche 7 bis 9, wobei die Kohlefaserumwicklung (30) einen Außendurchmesser aufweist, der dem Außendurchmesser des Kupplungsabschnitts (25) entspricht.

11. Aufreibvorrichtung nach einem der Ansprüche 1 bis 10, wobei die Außenfläche (23) des Schnittstellenelementanbringungsabschnitts eine Aussparungsstruktur (24) umfasst.

12. Aufreibvorrichtung nach Anspruch 11, wobei die Aussparungsstruktur (24) eine Umfangsnut umfasst.

13. Aufreibvorrichtung nach einem der Ansprüche 11 oder 12, wobei die Aussparungsstruktur (24) eine Rändelfläche umfasst.

14. Aufreibvorrichtung nach einem der Ansprüche 1 bis 13, wobei das Schnittstellenelement (20) aus einer Titanlegierung hergestellt ist.

15. Aufreibvorrichtung nach einem der Ansprüche 1 bis 14, wobei sowohl die Welle (10) als auch das Schnittstellenelement (20) jedes eine gestreckte Durchgangsbohrung (19, 29) aufweisen, wobei die beiden Durchgangsbohrungen aneinander ausgerichtet sind.

## Revendications

1. Dispositif d'alésage comportant :
une tige (10) avec une partie de montage (11), la partie de montage ayant une face d'extrémité (12) et une surface extérieure (13),
un élément d'interface (20) pour le couplage mécanique d'un dispositif externe, l'élément d'interface comportant une partie de montage (21), la partie de montage ayant une face d'extrémité (22) et une surface extérieure (23),
un enveloppement de fibres de carbone (30),
dans lequel la face d'extrémité (12) de la partie de montage de tige et la face d'extrémité (22) de la partie de montage d'élément d'interface sont dirigées l'une vers l'autre,
**caractérisé en ce que** l'enveloppement de fibres de carbone s'étend à la fois sur la surface extérieure (13) de la partie de montage de tige et la surface extérieure (23) de la partie de montage d'élément d'interface.

2. Dispositif d'alésage selon la revendication 1, dans lequel la tige (10) est constituée d'un matériau renforcé par des fibres de carbone.

3. Dispositif d'alésage selon l'une quelconque des revendications 1 et 2, dans lequel la face d'extrémité (12) de la partie de montage de tige et la face d'extrémité (22) de la partie de montage d'élément d'interface viennent en butée l'une contre l'autre.

4. Dispositif d'alésage selon l'une quelconque des revendications 1 à 3, dans lequel la partie de montage de tige (11) et la partie de montage d'élément d'interface (21) ont des diamètres extérieurs correspondants.

5. Dispositif d'alésage selon l'une quelconque des revendications 1 à 4, dans lequel l'enveloppement de fibres de carbone (30) comporte un couvercle de moule (35).

6. Dispositif d'alésage selon la revendication 5, dans lequel le couvercle de moule (35) est un couvercle de moule de thermofixation.

7. Dispositif d'alésage selon l'une quelconque des revendications 1 à 6, dans lequel l'élément d'interface (20) comporte une partie de couplage (25) pour une tête d'alésoir en tant que dispositif externe.

8. Dispositif d'alésage selon la revendication 7, dans lequel la partie de couplage (25) comporte une queue d'aronde (26).

9. Dispositif d'alésage selon l'une quelconque des revendications 7 et 8, dans lequel une surface extérieure (27) de la partie de couplage et la surface extérieure (23) de la partie de montage d'élément d'interface sont coaxiales, dans lequel la surface extérieure (23) de la partie de montage d'élément d'interface recule sur la surface extérieure (27) de la partie de couplage.

10. Dispositif d'alésage selon l'une quelconque des revendications 7 à 9, dans lequel l'enveloppement de fibres carbone (30) a un diamètre extérieur correspondant à un diamètre extérieur de la partie de couplage (25).

11. Dispositif d'alésage selon l'une quelconque des revendications 1 à 10, dans lequel la surface extérieure (23) de la partie de montage d'élément d'interface comporte une structure d'évidement (24).

12. Dispositif d'alésage selon la revendication 11, dans lequel la structure d'évidement (24) comporte une rainure circonférentielle.

13. Dispositif d'alésage selon l'une quelconque des revendications 11 et 12, dans lequel la structure d'évidement (24) comporte une surface moletée.

14. Dispositif d'alésage selon l'une quelconque des revendications 1 à 13, dans lequel l'élément d'interface (20) est constitué d'un alliage de titane.

15. Dispositif d'alésage selon l'une quelconque des revendications 1 à 14, dans lequel à la fois la tige (10) et l'élément d'interface (20) ont chacun un trou traversant allongé (19, 29), les deux trous traversants s'alignant l'un avec l'autre.
